# FASCICULE DE BREVET EUROPEEN

(11) **EP 2 124 731 B1**
(45) Date de publication et mention de la délivrance du brevet: **27.06.2012**
(21) Numéro de dépôt: 08708422.4
(22) Date de dépôt: 30.01.2008
(51) Int. Cl.: A61B 5/0205, A61B 5/024, G08B 29/18

(54) **DISPOSITIF DE MESURE D'AU MOINS UN PARAMETRE PHYSIOLOGIQUE**
VORRICHTUNG ZUR MESSUNG MINDESTENS EINES PHYSIOLOGISCHEN PARAMETERS
DEVICE FOR MEASURING AT LEAST ONE PHYSIOLOGICAL PARAMETER

(30) Priorité: 06.02.2007 FR 0700841
(43) Date de publication de la demande: 02.12.2009
(73) Titulaire: Université de Rennes 1, 35065 Rennes Cedex (FR); Université de Bretagne Sud, 56321 Lorient (FR)
(72) Inventeur: GOUJON, Jean-Marc, F-22300 Lannion (FR); GUYADER, Patrick, F-22300 Lanmerin (FR); THUILLIER, Sandrine, F-56100 Lorient (FR); BILLON, Michel, F-22300 Lannion (FR); L'HER, Henry, F-22730 Tregastel (FR); ROCHARD, Philippe, F-22700 Louannec (FR); LE PAGE, Ronan, 22300 Lannion (FR)
(74) Mandataire: Bioret, Ludovic
(86) Numéro de dépôt international: PCT/EP2008/051107
(87) Numéro de publication internationale: WO 2008/107238

(56) Documents cités:
- EP-A- 1 615 187
- WO-A-01/17421
- WO-A-02/30277
- DE-A1- 3 345 739
- DE-C2- 19 531 479
- FR-A1- 2 808 609
- US-A- 4 178 916
- US-A- 5 494 043

## Description

### 1. Domaine de l'invention

Le domaine de l'invention est celui de la mesure de paramètres physiologiques chez un sujet vivant, tels que, par exemple, le pouls, la saturation du sang en oxygène, l'impédance de la peau, la tension artérielle, le rythme respiratoire, l'arythmie respiratoire, la température cutanée, le taux de sudation ou le débit sanguin. Une telle mesure a notamment pour but d'aider à connaître l'état (santé, inconfort, ...) d'un sujet vivant.

Plus précisément, l'invention s'applique aux systèmes de mesure non invasifs et ambulatoires, portés au poignet.

L'invention peut trouver des applications dans de nombreuses situations, et peut notamment équiper des personnes âgées et/ou à mobilité réduite, des travailleurs isolés, des enfants, ou même des actifs.

### 2. Art antérieur

Les paramètres physiologiques tels que le pouls, les paramètres respiratoires, la tension artérielle, etc. sont souvent un reflet de la santé, ou tout du moins du confort d'un sujet vivant. Différents capteurs sont connus pour obtenir des mesures ces paramètres physiologiques.

### 2.1- La saturation du sang en oxygène

La saturation du sang en oxygène (qui est représentative notamment de la qualité de la respiration) peut être mesurée au moyen d'oxymètres (également appelés oxymètres de pouls) qui permettent de mesurer la quantité d'oxygène dissoute dans le sang.

Un oxymètre de pouls comprend classiquement au moins deux sources lumineuses ayant un rayonnement autour de 600 nm pour l'une, et de 940 nm pour l'autre. Un signal traversant ou rétrodiffusé est capté en fonction de l'absorption différentielle du sang. Un algorithme détermine alors la part de l'hémoglobine oxydée, une désaturation de l'hémoglobine étant synonyme de danger pour le sujet.

Cependant, la mise en oeuvre en continu des techniques connues d'oxymétrie est consommatrice d'énergie, et n'est donc généralement pas utilisable dans le cas de systèmes de mesure ambulatoire. De plus, elle est sensible aux mouvements du sujet.

Aussi, pour économiser l'énergie consommée par le système optique de l'oxymètre de pouls, une stratégie consiste à surveiller l'onde mécanique sur laquelle se superpose une modulation d'amplitude et de fréquence due à la respiration. Il est en effet plus avantageux de mesurer et démoduler l'onde de pression mécanique. Sauf dans le cas d'apnées obstructives, si cette modulation est présente, on peut supposer que l'oxygénation du sujet est correcte. Dans ce cas, il est inutile d'aller déclencher une mesure optique de l'oxygénation. Lorsque la modulation due à la respiration n'est pas perceptible, on déclenche alors une mesure optique, plus consommatrice en énergie.

Dans ce contexte de surveillance de l'onde mécanique, en utilisant une diode électroluminescente (encore appelée LED pour « Light Emitting Diode »), il est possible de rétablir le profil d'écoulement du sang, et de déduire ainsi la période entre deux impulsions.

### 2.2- Impédance de la peau

L'impédance de la peau est un autre paramètre physiologique utile pour déterminer l'état de santé ou d'inconfort chez un sujet vivant.

Lorsque le stress d'un individu augmente anormalement, il développe une réaction d'alarme, encore appelée réponse de fuite ou de combat. Elle est initiée au moyen d'hormones émises par le cerveau soumis au stress, et se traduit généralement par :
- une diminution de la régularité du rythme cardiaque ;
- une augmentation de la pression artérielle ;
- une variation notable des paramètres respiratoires ;
- des transferts d'énergie entraînant une sudation très importante à l'intérieur des mains ou sous les pieds.

Aussi, l'effet de ces symptômes sur un sujet peut être évalué en surveillant en continu l'impédance de la peau. Cette mesure (également appelée mesure de la résistance électrodermale) représente la résistance dermique (tel qu'expliqué dans le document intitulé « Bioelectromagnetism. Principles and applications of bioelectric and biomagnetic fields », chapitre 27, publié en 1995 aux éditions Oxford University Press). Elle est réalisée au moyen de deux électrodes EDR (pour « ElectroDermal Response »).

La cause de variation de l'impédance de la peau est la sudation. Les deux endroits les plus sensibles pour mesurer cette impédance sont la voûte plantaire et l'intérieur de la main, mais il s'avère peu acceptable (dans le cas d'un port en continu) de positionner des capteurs à ces endroits. La mesure de la sudation est effectuée à l'intérieur du poignet, qui est également un lieu de passage des flux sanguins et électriques.

### 2.3- Le pouls

Le calcul du pouls d'un sujet permet également de déterminer l'état de santé d'un individu. Il peut être déterminé à partir de la mesure :
- d'une onde mécanique, dans le cas de systèmes de mesure ambulatoires, le capteur d'ondes mécaniques peut être placé par exemple dans un boîtier-montre tenu par un bracelet autour du poignet. La mesure de l'onde mécanique peut également être effectué par le touché du poignet sur l'artère radiale ;
- d'une onde pléthysmographique, pour des applications oxymétriques, il est par exemple possible d'utiliser un capteur pléthysmographique placé au bout de l'index doigt ou au lobe de l'oreille, ce qui s'avère généralement inconfortable ;
- d'une onde électrique, l'onde électrique est mesurée par des électrodes ECG (pour « Electro CardioGramme »), maintenues soit collées sur la peau soit par un bandeau sur la poitrine du sujet, qui permettent de déterminer l'activité électrique du coeur.

Il est également possible de calculer la variabilité cardiaque, en temps réel, qui est plus représentative d'une situation modélisée possible.

### 2.4- La température cutanée

La température cutanée d'un sujet se révèle être également un paramètre physiologique permettant de déterminer l'état de santé d'un sujet. Elle est conventionnellement mesurée par un capteur de température à base de semi-conducteur positionné à l'intérieur du poignet.

### 2.5- Le débit sanguin

Les mesures pléthysmographiques permettent d'estimer le débit (ou flot) sanguin en mesurant l'augmentation du volume des vaisseaux sanguins, lors de l'occlusion du retour veineux. Elles sont effectuées par rétrodiffusion optique sur une artère, par exemple l'artère radiale. Les mesures pléthysmographiques sont souvent préalables à une mesure d'oxymétrie.

### 2.6- Dispositifs non invasifs

D'une façon générale, les techniques et les dispositifs de mesure non invasifs et ambulatoires connus (par exemple le bracelet de mesure décrit dans la demande de brevet français n°FR 0005822 publiée comme FR 2 808 609 A1), permettant de mesurer certains des différents paramètres physiologiques précités au moyen de capteurs physiologiques, présentent l'inconvénient majeur de fournir des signaux de mesure qui sont souvent très bruités.

En effet, les mouvements relatifs d'un bracelet de mesure classique (par exemple de type boîtier montre) par rapport au poignet du sujet génèrent un bruit, dit bruit de mouvement, sur les signaux de mesure, ce qui réduit le rapport signal sur bruit des signaux de mesure. Par ailleurs, l'éloignement des capteurs physiologiques de la zone mesurée (par exemple une artère) réduit également le rapport signal sur bruit des signaux de mesure.

Ainsi, la qualité des signaux mesurés n'est généralement pas suffisante pour fournir une mesure optimale des paramètres physiologiques du sujet. La mesure de ces paramètres est dès lors plus aléatoire et imprécise. Ainsi, les dispositifs de l'art antérieur ne fournissent pas des mesures fiables et précises dans certaines conditions.

Pour remédier à cet inconvénient, on connaît, dans l'art antérieur, des dispositifs de mesure de quantités physiologiques fixés au poignet (de type montre) qui ont aussi une protubérance pour assurer le contact avec la peau au niveau du poignet. Par exemple, le document WO 02/30277 A2 décrit un tel dispositif pour mesurer la tension artérielle. Ce dispositif comporte une protubérance qui, lorsque ce dispositif est porté par l'utilisateur, se trouve près de l'artère radiale. Le document DE 1 95 31 479 C2 décrit un dispositif similaire qui est apte à mesurer la fréquence cardiaque de l'utilisateur. Ce dispositif a une protubérance demi-cylindrique. Un dispositif de ce type est aussi décrit dans le document US 2003/212335 A1 ; pourtant, ce dispositif ne se prête pas à l'utilisation ambulatoire. Finalement, le document DE 33 45 739 A1 décrit un dispositif sous forme d'une montre. Ce dispositif peut mesurer la fréquence cardiaque et la tension artérielle. Dans un mode de réalisation, il comporte aussi une protubérance. De plus, ce dispositif a des capteurs supplémentaires qui produisent des signaux utiles pour le débruitage.

Bien que ces dispositifs permettent des mesures physiologiques plus fiables, il serait toujours désirable d'améliorer la fiabilité du signal et la détermination de celle-ci.

### 3. Objectifs de l'invention

L'invention, dans au moins un mode de réalisation, a notamment pour objectif de pallier ces différents inconvénients de l'état de la technique.

Plus précisément, l'un des objectifs de la présente invention, dans au moins un mode de réalisation, est de fournir un dispositif de mesure d'au moins un paramètre physiologique à base d'au moins un capteur physiologique qui permette d'augmenter le rapport signal à bruit des signaux de mesure issus du ou des capteurs physiologiques.

En particulier, l'invention a pour objectif de fournir, dans au moins un mode de réalisation, un tel dispositif de mesure qui permette de réduire le bruit sur les signaux de mesure, dû aux mouvements relatifs du dispositif par rapport au poignet et dû à l'éloignement entre les capteurs physiologiques et l'artère radiale du poignet.

Un autre objectif de l'invention, dans au moins un mode de réalisation, est de fournir un tel dispositif qui permette de mesurer plusieurs paramètres physiologiques.

Un autre objectif de l'invention, dans au moins un mode de réalisation, est de fournir un tel dispositif qui soit fiable et simple à mettre en oeuvre.

Par ailleurs, un objectif complémentaire de l'invention, dans au moins un mode de réalisation, est de fournir un tel dispositif :
- qui soit relativement simple et peu coûteux à fabriquer ;
- qui présente une faible consommation énergétique, et donc une bonne autonomie ;
- qui soit peu encombrant, et ergonomique (c'est-à-dire qui ne présente pas de gêne pour le porteur).

### 4. Exposé de l'invention

Ces objectifs, ainsi que d'autres, sont atteints selon l'invention à l'aide d'un dispositif de mesure d'au moins un paramètre physiologique chez un sujet vivant, tel que défini par les caractéristiques de la revendication 1.

Le principe de l'invention consiste donc à mettre en oeuvre, sur un module de mesure, au moins une protubérance afin de stabiliser, grâce à la protubérance, le dispositif et en particulier son module de mesure par rapport au poignet du porteur, ce qui permet ainsi de réduire le bruit sur le ou les signaux issu du ou des capteur(s) dû aux mouvements relatifs du dispositif par rapport au poignet. Cette protubérance porte au moins deux capteurs mesurant des informations différentes. Ceci permet de contrôler de façon précise et efficace le placement de ces capteurs, par rapport à l'artère radiale du poignet.

Cette technique permet également d'approcher le ou les capteur(s) physiologique(s) de la protubérance (ou palpeur) au plus près de l'artère radiale du poignet du sujet ce qui permet de réduire le bruit dû à l'éloignement entre le ou les capteurs et l'artère.

Ainsi les variations de pression de l'artère radiale sont transmises de façon optimisée au détecteur.

Bien entendu, le dispositif selon l'invention comprend un ou plusieurs capteur(s) disposé(s) à proximité de la protubérance .

Le dispositif de l'invention permet par exemple de prendre en compte un ou plusieurs paramètres physiologiques, ce qui peut permettre de :
- évaluer le stress du sujet vivant ;
- détecter un malaise du sujet (ceci est rendu possible grâce à l'amélioration du rapport signal utile sur signal de bruit qu'apporte le dispositif de l'invention par rapport aux dispositifs classiques, et la mise en oeuvre de méthodes de traitement du signal correspondantes) ;
- effectuer de la pléthysmographie ambulatoire.

Préférentiellement, ladite protubérance est montée mobile par rapport audit boîtier de mesure.

Selon un mode de réalisation avantageux de l'invéntion, au moins un desdits capteurs de mesure est un capteur de mesure passif qui réalise des mesures en continu. Selon un autre aspect avantageux de l'invention, un desdits capteurs de mesure est un capteur d'ondes de pression piézo-électrique.

Ce capteur piézo-électrique peut notamment comprendre deux cellules piézoélectriques montées en opposition dans un plan tangent au module de mesure.

On peut par exemple câbler les deux cellules piézo-électriques montées en opposition dans un plan tangent au module de mesure au moyen de bandes conductrices collées.

Ceci permet notamment de minimiser les effets de bougé.

Notamment, un desdits capteurs de mesure peut être un oxymètre optique mettant en oeuvre au moins une première et une seconde sources lumineuses, émettant respectivement à des première et seconde longueurs d'onde, et au moins un photodétecteur.

Ainsi, on peut réaliser de la pléthysmographie ambulatoire à deux longueurs d'onde par réflexion (ou rétrodiffusion) optique au niveau de l'artère radiale afin par exemple de mesurer la saturation du sang en oxygène du sujet (ou oxymétrie) ou la part d'hémoglobine oxydée du sujet.

Selon un mode de réalisation particulier, ladite protubérance comprend au moins deux électrodes de mesure d'une résistance électrodermale.

Dans ce cas, lesdits moyens de traitement comprennent avantageusement des moyens de redressement, de filtrage passe bande et d'amplification d'au moins un signal issu desdites électrodes.

Ainsi, les moyens de redressement peuvent par exemple être constitués d'une diode et d'une capacité pour effectuer une détection de crête.

Lesdits moyens de traitement comprennent également préférentiellement des moyens de suppression d'une composante continue dudit au moins un signal issu des électrodes.

Préférentiellement; lesdits moyens de traitement délivrent au moins une information représentative d'au moins un paramètre physiologique appartenant au groupe comprenant :
- le pouls ;
- le taux d'oxygène dans le sang ;
- l'impédance de peau ;
- une grandeur fonction de l'impulsion cardiaque;
- le rythme respiratoire, l'arythmie respiratoire et/ou l'amplitude respiratoire ;
- la température cutanée ;
- la sudation au niveau du poignet ;
et les variations et/ou combinaisons de ces grandeurs.

Avantageusement, le dispositif de mesure comprend des moyens de déclenchement d'une alarme lorsque ledit au moins un capteur constate une anomalie appartenant au groupe suivant :
- absence d'énergie au niveau desdits capteurs d'ondes de pression piézoélectriques ;
- impédance supérieure à un seuil prédéterminé desdites électrodes de mesure de la résistance électrodermale ;
- variation supérieure à un seuil prédéterminé de la température indiquée par ledit au moins un capteur de température ;
- absence de modulation de flux indiqués par ledit au moins photodétecteur.

### 5. Listes des figures

D'autres caractéristiques et avantages des modes de réalisation de l'invention apparaîtront à la lecture de la description suivante d'un exemple illustrant l'invention , donné à titre d'exemple indicatif et non limitatif (tous les modes de réalisation de l'invention ne sont pas limités aux caractéristiques et avantages de ce mode de réalisation préférentiel), et des dessins annexés, dans lesquels :
- les figures 1A et 1B présentent des schémas d'un dispositif de mesure d'au moins un paramètre physiologique chez un sujet vivant en perspective (figure 1A) et vu de face (figure 1B);
- la figure 2 présente une coupe transversale schématique au niveau du poignet d'un individu ;
- les figures 3A et 3B présentent une vue de dessus et une vue de côté d'un schéma du module de mesure ;
- la figure 4 présente un schéma de principe du palpeur du module de mesure ;
- la figure 5 présente un organigramme des étapes principales d'un algorithme de traitement de l'onde mécanique de pression générée par l'artère radiale ;
- la figure 6 présente un circuit électronique de traitement de l'onde mécanique de pression ;
- la figure 7 présente un organigramme des étapes principales d'un algorithme de détermination de l'impédance de peau du sujet ;
- la figure 8 présente un schéma d'un circuit électronique de détermination de l'impédance de peau ;
- la figure 9 présente un schéma d'un circuit électronique de détermination de l'impédance de peau selon une variante du dispositif décrit par les figures précédentes.

### 6. Description détaillée

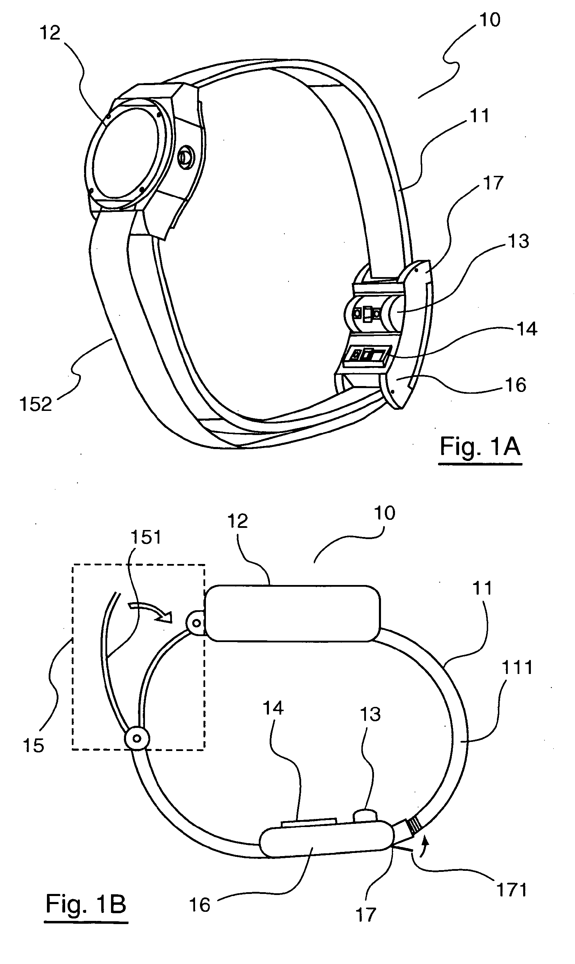

On décrit ci-après, en relation avec les ***figures 1A et 1B***, les schémas d'un dispositif de mesure 10 d'au moins un paramètre physiologique chez un sujet vivant en perspective (figure 1A) et vu de face (figure 1B).

Ainsi, préférentiellement, le dispositif de mesure 10 est un bracelet de mesure.

Le dispositif de mesure 10 comprend notamment un module de mesure 16 comprenant des moyens de calage présentant une protubérance 13 faisant saillie à l'intérieur du bracelet afin de caler le module de mesure au niveau d'un poignet du sujet, la protubérance (ci-après désignée par palpeur) comprenant au moins un capteur physiologique. Bien entendu, selon une variante du dispositif, on peut envisager un module de mesure 16 comprenant au moins deux protubérances distinctes.

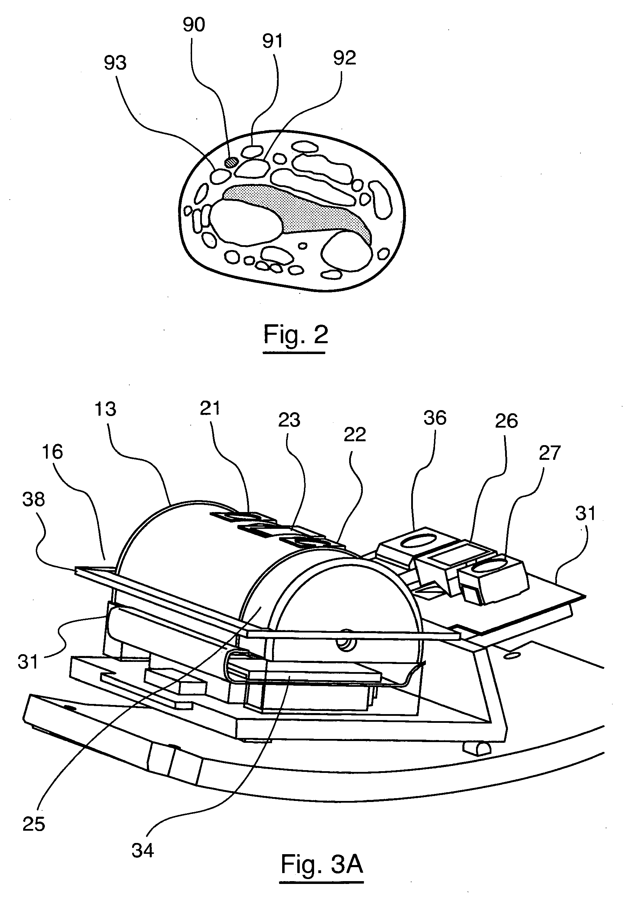

Dans ce cas, la protubérance (ou palpeur ) 13, dont la partie supérieure est par exemple de forme sensiblement demi-cylindrique, est prévue pour se positionner de manière stable dans la partie molle du poignet du sujet située à proximité de l'artère radiale 90 qui est entourée de tendons 91, 92, 93, tels qu'illustrés sur la ***figure 2******.*** Ainsi, la forme de la protubérance est définie de façon à s'inscrire entre deux tendons du poignet. Par ailleurs, la forme demi-cylindrique du palpeur 13 permet également de minimiser la sensation d'inconfort d'un utilisateur lors d'un port prolongé du bracelet de mesure en épousant la partie molle du poignet.

Ainsi, le bracelet de mesure 10 permet par exemple, grâce à la protubérance, de stabiliser le module de mesure par rapport au poignet, ce qui permet de réduire le bruit sur le ou les signaux issu du ou des capteur(s) physiologique(s) dû aux mouvements relatifs du bracelet par rapport au poignet.

Il permet également d'approcher le ou les capteur(s) physiologique(s) de la protubérance (ou palpeur) au plus près de l'artère radiale du poignet du sujet ce qui permet de réduire le bruit dû à l'éloignement entre le ou les capteurs et l'artère.

Le bracelet de mesure 10 conforme à l'invention est plus fiable et plus précis que les bracelets de mesure classiques, en particulier parce qu'il réduit le bruit présent sur les signaux de mesure, par un positionnement précis des capteurs, et une absence de mouvements de ceux-ci.

Les capteurs physiologiques du présent bracelet de mesure 10 peuvent par exemple délivrer au moins un signal utile représentatif d'au moins un paramètre physiologique appartenant au groupe comprenant notamment :
- l'onde de pression mécanique, avec laquelle on peut déduire le pouls et la fréquence respiratoire ;
- la saturation du sang en oxygène ;
- l'impédance de peau ;
- une grandeur fonction de la tension artérielle ;
- le rythme respiratoire, l'arythmie respiratoire et/ou l'amplitude respiratoire ;
- la température cutanée ;
- la sudation localisée sous le bracelet de mesure au niveau du poignet.

Le fait que l'ensemble des capteurs soit intégré dans le module de mesure du bracelet de mesure permet d'obtenir une miniaturisation du système de mesure de paramètres physiologiques.

### 6.1- Architecture du bracelet de mesure

Tel qu'illustré par les figures 1A et 1B, le bracelet de mesure 10 comprend notamment :
- un bracelet de maintien 11 qui comprend également une partie souple 111 (mais de préférence non élastique) par exemple fabriquée en élastomère 61 shores ;
- un module 16 de mesure qui peut comprendre un palpeur 13 dans lequel sont intégrés un premier ensemble de capteurs physiologiques ci-après décrits et un second ensemble 14 de capteurs physiologiques externes au palpeur, le palpeur 13 s'étendant sur une majeure partie de la largeur du module 16, sensiblement perpendiculairement à l'axe défini par le bracelet 11 ;
- un système 12 de traitement et d'affichage d'informations obtenues notamment à partir des mesures des paramètres physiologiques ou de toute variation et/ou combinaison de ces grandeurs ;
- un système de fixation et/ou de serrage 15 par exemple à base d'une bande d'attache 151 (par exemple une bande VELCRO, marque déposée) ;
- un système de réglage 17 réversible de la longueur de la partie souple 111 entre le système de traitement et d'affichage 12 et le module de mesure 16 permettant notamment de positionner au mieux le palpeur 13 au niveau du poignet de l'utilisateur. Ainsi, le système de réglage 17 permet, par exemple, de faire pénétrer une partie du bracelet 11 à l'intérieur du module de mesure 16 réduisant ainsi la longueur de la partie souple 111 entre le système 12 et le module de mesure 16. Lorsque l'utilisateur considère que la position du palpeur est optimale (et donc la longueur de la partie souple 111), il peut verrouiller cette position au moyen d'une attache 171 (appartenant au système de réglage 17). De manière optionnelle, et tel qu'illustré par la figure 1A, de préférence le bracelet de mesure 11 peut comprendre une bande 152 de circuit imprimé souple permettant d'assurer la transmission de signaux électriques entre le module de mesure 16 et le système 12 de traitement et d'affichage, par exemple pour permettre l'affichage de la valeur de l'impédance de peau de l'utilisateur.

Dans ce cas, ces moyens de liaison sont par exemple intégrés dans la structure même du bracelet 11.

De plus, le module de mesure 16 peut avoir la forme d'un boîtier de mesure 16, tel qu'illustré sur la figure 1A.

Alternativement, le module de mesure 16 peut être intégré à la partie souple 111 du bracelet 11.

Selon un autre variante du dispositif, on peut envisager l'intégration du système 12 de traitement et d'affichage d'informations au sein du module de mesure 16.

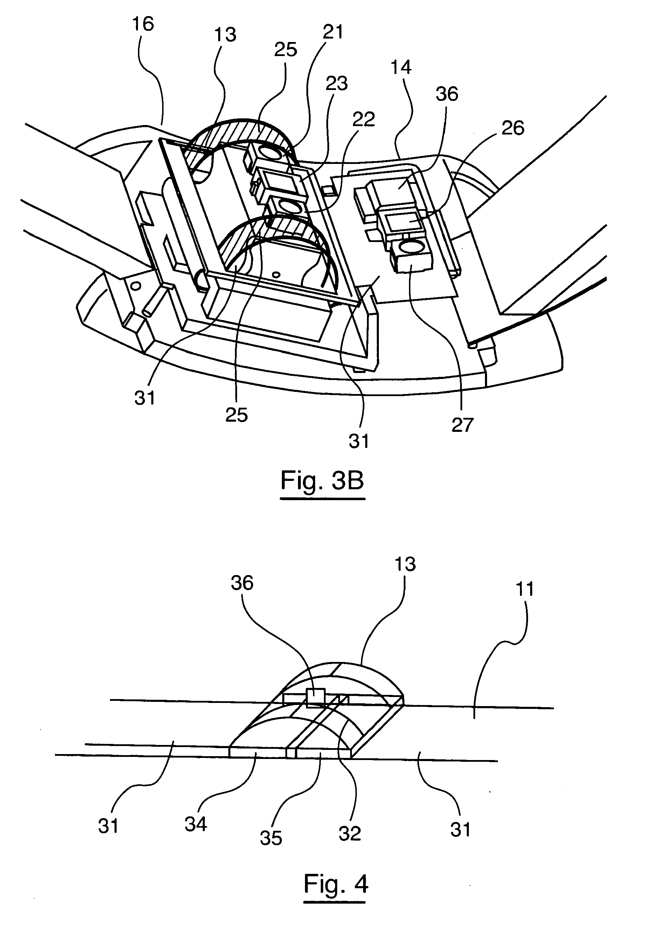

On présente, en relation avec les ***figures 3A*** ***et*** ***3B******,*** un schéma du module de mesure 16, mentionné ci-dessus.

Le module de mesure 16 comprend par exemple un premier ensemble de mesure (qui est le palpeur 13 comprenant le premier ensemble de capteurs) et un second ensemble de mesure (constitué par le second ensemble de capteurs 14).

Ainsi le palpeur 13 (ou premier ensemble de mesure) peut par exemple comprendre :
- une première diode électroluminescente 21 dont la longueur d'onde est, par exemple, voisine de 660 nm :
- une seconde diode électroluminescente 22 dont la longueur d'onde est, par exemple, voisine de 940 nm ;
- une photodiode 23 (encore appelée photodétecteur) ;
- deux électrodes 25 de mesure de résistance électrodermale (ou électrodes EDR pour « ElectroDermal Response ») qui sont des électrodes sèches par exemple espacées de 4 mm, chacune longue de 10 mm et large de 4 mm, et qui peuvent présenter des contacts surfaciques dorés ;
- un capteur (ou sonde) de température 36 par exemple associé à un amplificateur électrique. Le capteur de température peut par exemple être de type LM35 commercialisé par la société NATIONAL SEMICONDUCTOR (marque déposée). On considère dans la suite que l'amplificateur est compris dans le capteur de température. Bien entendu, l'amplificateur peut être externe au capteur de température.

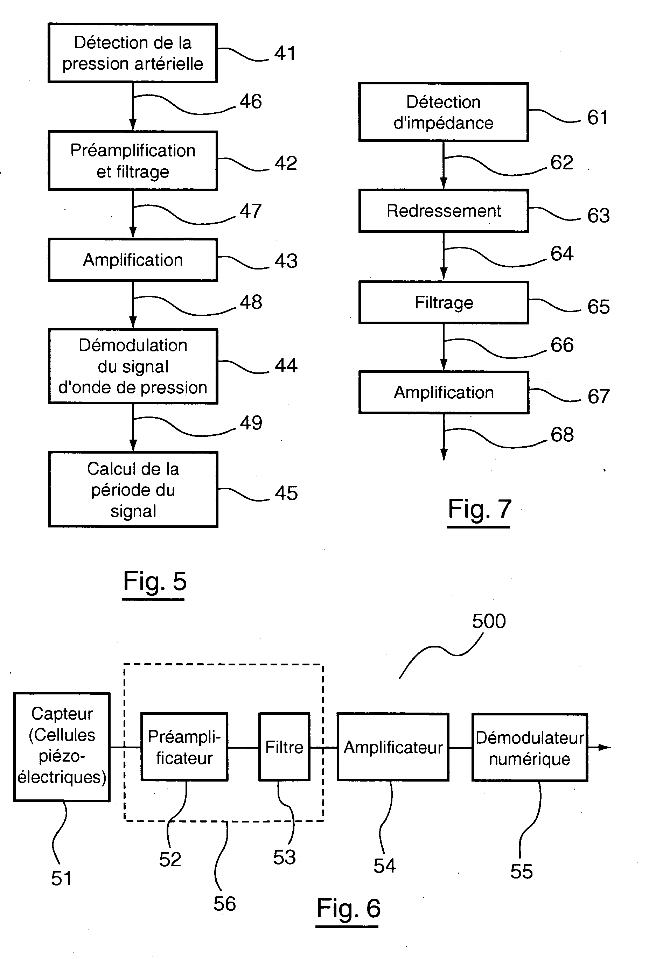

Par ailleurs, le premier ensemble de mesure peut comprendre des moyens de filtrage des signaux de mesure du premier ensemble de capteurs physiologiques comprenant un filtre 53 passe-bande (ci-après illustré sur la figure 5) réalisé notamment à partir d'une résistance et d'une capacité. Par exemple, la bande passante du filtre passe bande 53 est comprise entre 0,1 et 3 Hz.

Les diodes électroluminescentes 21 et 22 et la photodiode 23 constituent des premiers capteurs optiques qui peuvent former par exemple un premier oxymètre optique. Ainsi, la protubérance peut porter au moins un oxymètre optique.

Par ailleurs, tel qu'illustré par la ***figure 4*** (présentant plus précisément le schéma du palpeur 13), le palpeur 13 peut également comprendre un capteur d'onde de pression (sensible à la variation de la tension artérielle du sujet) réalisé au moyen de cellules piézoélectriques 34 et 35 situées sur la partie inférieure plane du palpeur 13. Les deux cellules piézoélectriques sont, par exemple, du type PXE52 commercialisé par la société PHILIPS (marque déposée), et dont les dimensions sont par exemple 10 mm pour la longueur, 4 mm pour la largeur et 0,5 mm pour l'épaisseur.

Bien entendu, dans des variantes de cet exemple, on peut mettre en oeuvre dans le palpeur 13 des cellules piézo-électriques de caractéristiques et de dimensions différentes.

Les deux cellules piézo-électrique 34 et 35 (présentes à la fois sur la figure 3A et sur la figure 4) sont par exemple montées en opposition (tête-bêche) dans un plan tangent au bracelet pour que les signaux provoqués par les contraintes latérales s'annulent, permettant ainsi de minimiser les effets de « bougé ». Elles peuvent être posées sur un matériau conducteur rigide qui assure leur protection (par exemple un clinquant métallique en cuivre de 0,5 mm d'épaisseur) et fiabilise le montage.

Le matériau conducteur rigide peut être soudé sur un circuit imprimé 31. Ce circuit imprimé 31 peut être un circuit souple conducteur (réalisé par exemple en Kapton) de faible épaisseur (par exemple quelques dizaines de microns). Il peut être rapporté contre la partie souple 111 du bracelet 11, et s'étendre le long de la partie souple 111.

Les premier et second ensembles de mesure peuvent être disposés sur une des deux surfaces du circuit imprimé 31. Bien entendu selon une variante du dispositif décrit ci-dessus, d'autres composants (par exemple un amplificateur, des moyens de traitement numérique, ...) peuvent être soudés sur au moins une des deux surfaces du circuit imprimé 31.

Selon une variante dudit dispositif, on peut envisager que le circuit imprimé 31 soit constitué de deux circuits imprimés distincts solidaires l'un de l'autre par exemple par un contact électrique. Selon une autre variante dudit dispositif, le circuit imprimé 31 peut être réduit à un simple fil.

Par ailleurs, les cellules piézo-électriques peuvent être recouvertes par un matériau conducteur souple de faible épaisseur (par exemple quelques microns) jouant le rôle de contact électrique. Ainsi en recouvrant l'ensemble de la surface de chacune des cellules piézo-électriques, le matériau conducteur souple permet de maintenir la conduction électrique lorsqu'une des cellules piézo-électriques se casse.

Ce contact électrique constitue l'entrée d'un préamplificateur 52 transformateur d'impédance (ci-après illustré sur la figure 5).

Le palpeur 13 est par exemple situé droit au-dessus de l'artère radiale du poignet du sujet qui porte le dispositif de mesure et ainsi, chaque capteur du palpeur est situé au plus proche de l'artère qui est la source des signaux que l'on souhaite mesurer. En conséquence, chaque capteur du palpeur 13 peut délivrer un signal de mesure (autrement appelé signal utile) présentant un rapport signal sur bruit amélioré.

La face inférieure plane du palpeur 13 peut par exemple être maintenue plaquée sur la face supérieure de chacune des cellules piézo-électriques par des moyens élastiques (par exemple des ressorts). Selon une variante du dispositif , la face inférieure plane du palpeur 13 peut être simplement collée sur la face supérieure des cellules piézo-électrique.

Une liaison glissière (non représentée) peut par exemple assurer le guidage vertical du palpeur par rapport à la surface supérieure des cellules piézo-électriques permettant de minimiser les frottements du palpeur 13 sur les contours d'un cadre 38 appartenant au boîtier extérieur 17 du module 16. Ainsi, la réception des contraintes mécaniques transmises à travers la peau du porteur au palpeur 13 (qui sont généralement de l'ordre de quelques dixièmes de Newton) est optimisée.

Alternativement, un joint étanche peut être intégré au boîtier extérieur 17 du module de mesure pour réaliser l'étanchéité entre le palpeur 13 et les contours du cadre 38.

Sous l'effet d'une onde de pression transmise par la peau du porteur utilisateur, le palpeur 13 (par exemple réalisé en matériau plastique rigide non déformable) transmet à son tour une contrainte de pression sur toute la surface des cellules piézoélectriques. Sous l'effet piézoélectrique direct, une charge est alors générée aux bornes des cellules piézoélectrique 34 et 35. Le préamplificateur 52 transformateur d'impédance transforme ensuite cette charge en tension.

Le second ensemble de capteurs 14 (disposé sur le circuit imprimé 31), dédié au débruitage des signaux utiles mesurés par les capteurs du premier ensemble de mesure, peut par exemple comprendre :
- une diode électroluminescente 27 dont la longueur d'onde est, par exemple, voisine de 660 nm ou de 940 nm. Bien entendu, selon une variante, le second ensemble de capteurs 14 peut comprendre plusieurs diodes électroluminescentes ;
- une photodiode 26.

Alternativement on peut envisager d'intégrer le capteur de température 36 au second ensemble de capteurs 14.

La diode électroluminescente 27 et la photodiode 26 constituent des seconds éléments optiques 26, 27 permettant d'isoler le bruit de mouvement.

Les seconds éléments optiques 26, 27 sont dédiés à la mesure de signaux de bruit quasiment pur destinés à être combinés aux signaux utiles (ou de mesure) issus des premiers capteurs optiques 21, 22, 23 afin de réduire l'influence du bruit sur ces signaux utiles.

### 6.2 Electronique du bracelet de mesure

La figure 5 présente un organigramme des étapes principales d'un algorithme de traitement de l'onde mécanique de pression générée par l'artère radiale et utilisant le signal utile issu du capteur de pression (constitué par les cellules piézoélectriques 34 et 35) selon l'exemple décrit ci-dessus. L'algorithme de traitement est mis en oeuvre au sein d'un circuit électronique de traitement 500 de l'onde mécanique de pression intégré au bracelet de mesure et illustré sur la figure 6.

Tel qu'illustré par la figure 6, le circuit électronique de traitement 500 de l'onde mécanique de pression peut par exemple comprendre :
- un module capteur 51 comprenant les deux cellules piézoélectriques 34 et 35 ;
- un module 56 comportant le préamplificateur 52 monté en série avec le filtre passe bande 53 ;
- un amplificateur 54 ; et
- un démodulateur numérique 55 (intégré par exemple dans sous forme d'un algorithme dans un microcontrôleur).

Tel qu'illustré par la figure 5, dans une étape 41 de détection de l'onde de pression artérielle, les contraintes exercées sur les cellules piézo-électriques 34 et 35 par les ondes mécaniques de pression générées par l'artère radiale (la source) peuvent être sommées afin d'obtenir un signal de pression 46.

Dans une étape 42, le signal de pression 46 est par exemple préamplifié et filtré grâce au bloc électronique 56 comprenant le préamplificateur 52 ainsi que le filtre passe bande 53 montés en série. On obtient ainsi le signal de pression préamplifié et filtré 47.

Dans une étape 43, le signal de pression préamplifié et filtré peut être amplifé (on obtient ainsi un signal de pression amplifié 48 sur lequel se superpose une modulation d'amplitude AM et de fréquence FM) puis est ensuite démodulé numériquement (on obtient ainsi un signal de pression démodulé 49), dans une étape 44, de façon à extraire les paramètres respiratoires (la fréquence respiratoire et l'arythmie éventuelle) du signal de pression amplifié 48.

Dans une étape 45, la période du signal de pression démodulé 49 est calculée. Cette période correspondant à la période cardiaque du sujet et est équivalente à l'intervalle R-R.

On obtient alors la localisation exacte de chaque pic du pouls, ce qui permet de reconstituer l'intervalle R-R. Une procédure de débruitage est éventuellement appliquée préalablement si le niveau de bruit repéré est jugé trop conséquent.

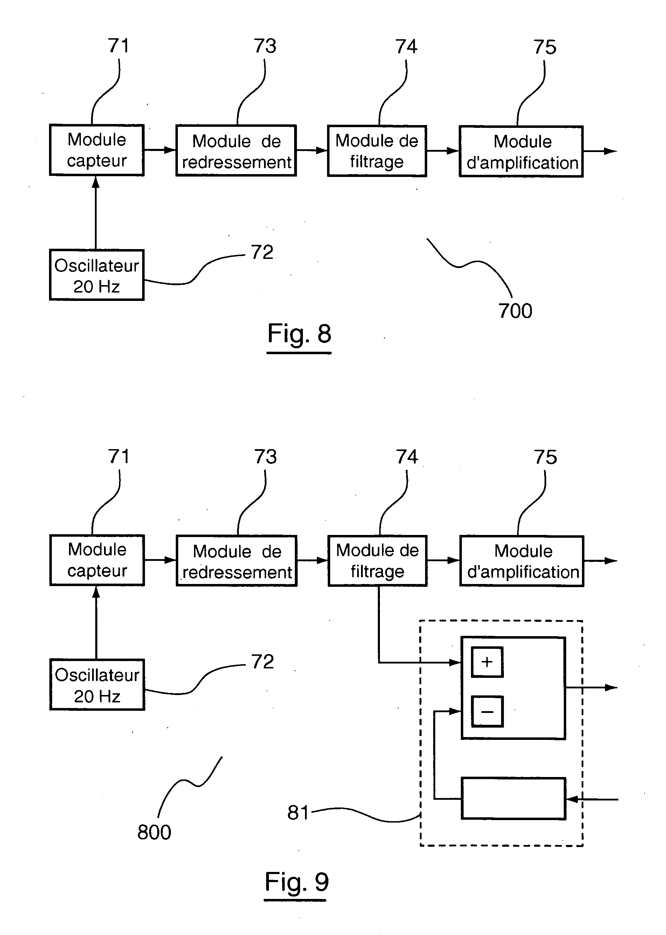

On présente, en relation avec la ***figure 7***, un organigramme des étapes principales d'un algorithme de détermination de l'impédance de la peau du sujet utilisant le signal utile issu des électrodes EDR 25 selon un exemple illustrant l'invention. L'algorithme de détermination est par exemple mis en oeuvre au sein d'un circuit électronique de détermination 700 de l'impédance de la peau intégré au bracelet de mesure et illustré sur la ***figure 8***.

Tel qu'illustré par la figure 8, le circuit électronique 700 comprend par exemple:
- un module capteur 71 comprenant les deux électrodes EDR 25 ;
- un oscillateur électrique 72 oscillant à une fréquence d'oscillation de 20Hz ;
- un module de redressement 73 ;
- un module de filtrage 74 ; et
- un module d'amplification 75.

Tel qu'illustré par la figure 7, dans une étape 61, le module capteur 71 peut mesurer un signal électrodermal 62 (signal utile issu des électrodes EDR 25) qui est proportionnel à l'impédance de la peau du sujet. Pour ce faire, on peut par exemple mettre en oeuvre un signal à la fréquence d'oscillation précitée issu de l'oscillateur électrique 72 (le signal valant quelques µA) pour éviter l'effet de polarisation des électrodes.

Dans une étape 63, le signal électrodermal 62 est par exemple redressé par un module de redressement 73 (par exemple une diode et une capacité effectuant classiquement une détection de crête). On obtient ainsi un signal électrodermal redressé 64.

Dans une étape 65, le signal électrodermal redressé 64 peut être filtré par un module de filtrage 74 (qui est par exemple un filtre passe bande dont la bande passante s'étend entre 0,1Hz et 2Hz) afin d'obtenir un signal électrodermal filtré 66 qui est ensuite amplifié (par exemple d'un facteur 10) grâce aux moyens d'amplification 75 lors d' une étape 67. On obtient ainsi un signal électrodermal amplifié 68.

Selon une variante de l'exemple précité (des figures 7 et 8), illustrée par la ***figure 9***, un circuit électronique 800 de détermination de l'impédance de peau peut comporter, outre les composants du circuit 700, une boucle de contre-réaction 81 supplémentaire permettant de supprimer la composante continue du signal électrodermal filtré 66.

Ainsi, deux signaux sont acquis. La composante continue du signal est restaurée, permettant d'obtenir la durée d'une situation de stress par exemple.

La température cutanée est obtenue à l'aide du capteur de température 36 après amplification par l'amplificateur du capteur de température 36. On peut ainsi obtenir la température cutanée à l'intérieur du poignet du sujet vivant avec une sensibilité de 110 mV/°C.

De préférence, les première 21 et seconde 22 diodes électroluminescentes associées à la photodiode 23 permettent notamment de réaliser des mesures pléthysmographiques à deux longueurs d'onde pour calculer le flot sanguin du sujet ou pour réaliser de l'oxymétrie afin de mesurer le taux d'oxygène dans le sang.

Ainsi, le bracelet de mesure, tel que décrit précédemment, minimise le bruit contenu dans les signaux de mesure en plaçant les capteurs physiologiques au plus près de l'artère radiale et en stabilisant le bracelet par rapport au poignet grâce à la protubérance ce qui peut permettre de gagner jusqu'à 20 dB par rapport aux mesures classiques.

Les seconds éléments optiques 26, 27 fournissent des signaux correspondant au bruit de mouvements du porteur (par exemple le bruit des tendons) et permettent de gagner jusqu'à 20 dB par rapport aux mesures classiques lorsqu'ils sont utilisés pour effectuer un débruitage optique.

Ainsi, le bracelet de mesure 11 permet notamment :
- une prise de mesures au plus près de la source (par exemple l'artère radiale) ;
- la mise en oeuvre d'un palpeur 13 permettant d'obtenir une réduction de la part de bruit des signaux mesurés par les capteurs physiologiques du bracelet (notamment le bruit d'artefact) ;
- la connaissance du système porté à partir de l'analyse de l'énergie délivrée par les cellules piézo-électriques 34 et 35, par les électrodes électrodermales 25, par le capteur 36 de température, ou par le retour des signaux optiques issus des premiers capteurs optiques ;
- la mesure de signaux issus des premiers capteurs optiques permettant le calcul de saturation en oxygène, validés par les signaux issus d'un capteur d'onde de pression ;
- la prise en compte de plusieurs paramètres physiologiques différents, permettant un traitement de données innovant ;
- la création d'un nouvel indice de stress.

Ainsi, les signaux optiques et mécaniques présentent des caractéristiques corrélées, garanties notamment par architecture du palpeur 13. En cas de doute sur la qualité d'un type de signal, il est possible de vérifier la qualité de l'autre.

Par ailleurs, le bracelet de mesure peut comprendre des moyens de génération d'une alarme locale et des moyens de transmission vers un site distant de ladite alarme, après un délai prédéterminé, lorsque l'alarme n'a pas été invalidée manuellement par le sujet.

Le bracelet de mesure peut également comprendre des moyens de déclenchement (par exemple manuels) émettant une alarme vers un site distant lorsque au moins un des capteurs constate une anomalie appartenant au groupe suivant :
- absence d'énergie au niveau des capteurs d'ondes de pression piézo-électriques ;
- impédance supérieure à un seuil prédéterminé (par exemple 1MégaΩ) des électrodes de mesure de la résistance électrodermale ;
- variation supérieure à un seuil prédéterminé (par exemple 1°C) de la température indiquée par le capteur de température ;
- absence de modulation de flux indiqués par au moins une photodiode.

Le bracelet de mesure peut comprendre des moyens d'affichage d'au moins une des informations appartenant au groupe comprenant :
- heure et date ;
- informations physiologiques ;
- alertes ;
- messages personnalisés, à un instant donné ;
- informations de localisation.

Le bracelet de mesure peut être utilisé dans le cadre de la détection de malaise, de la détection de chute, de la mesure de l'indice de stress avec ou sans feedback, de la détection de troubles respiratoires, etc...

Bien entendu, un bracelet de mesure peut effectuer des mesures d'autres paramètres physiologiques et comprendre pour ce faire d'autres capteurs physiologiques.

## Revendications

1. Dispositif de mesure (10) d'au moins un paramètre physiologique chez un sujet vivant, comprenant un module de mesure (16) monté sur un bracelet (11), ledit module (16) portant des moyens de traitement alimentés par au moins un signal issu d'au moins un capteur de mesure prévu pour venir en contact avec la peau dudit sujet, au niveau d'un de ses poignets,
ledit module de mesure (16) comprenant un premier ensemble de capteurs (21, 22, 23, 25, 34, 35, 36) portés par une protubérance (13) faisant saillie à l'intérieur dudit bracelet (11) et un deuxième ensemble de capteurs (14) situés à proximité de ladite protubérance (13), la forme de ladite protubérance (13) étant définie de façon à coopérer avec ledit poignet afin d'empêcher ou limiter une rotation dudit bracelet (11) autour dudit poignet et pour placer ledit premier ensemble de capteurs (21, 22, 23, 25, 34, 35, 36) et ledit deuxième ensemble de capteurs (14) au niveau de l'artère radiale dudit poignet, lesdits moyens de traitement comprenant des moyens de débruitage des signaux issus dudit premier ensemble de capteurs (21, 22, 23, 25, 34, 35, 36) en fonction des signaux issus dudit deuxième ensemble de capteurs (14),
**caractérisé en ce que** les capteurs dudit premier ensemble de capteurs comprennent au moins un capteur optique (21, 22, 23) et au moins un capteur d'ondes de pression (34, 35), de façon à effectuer des mesures basées sur des principes physiques différents et délivrant des signaux de mesure corrélés,
**en ce que** lesdits moyens de traitement sont alimentés par lesdits signaux de mesure corrélés issus dudit capteur optique (21, 22, 23) et dudit capteur d'ondes de pression (34, 35) dudit premier ensemble de capteurs.

2. Dispositif de mesure selon la revendication 1, **caractérisé en ce qu'**au moins un desdits capteurs de mesure dudit premier ensemble de capteurs (21, 22, 23, 25, 34, 35, 36) est un capteur de mesure passif qui réalise des mesures en continu.

3. Dispositif de mesure selon l'une des revendications 1 et 2, **caractérisé en ce que** ladite protubérance (13) est montée mobile par rapport audit module de mesure.

4. Dispositif de mesure selon l'une des revendications 1 à 3, **caractérisé en ce que** ledit capteur d'ondes de pression est un capteur piézo-électrique qui comprend deux cellules piézo-électriques (34, 35) montées en opposition dans un plan tangent au module de mesure (16).

5. Dispositif de mesure selon l'une des revendications 1 à 4, **caractérisé en ce qu'**un desdits capteurs de mesure dudit deuxième ensemble de capteurs (14) est un accéléromètre.

6. Dispositif de mesure selon l'une quelconque des revendications 1 à 5, **caractérisé en ce qu'**un desdits capteurs de mesure dudit premier ensemble de capteurs (21, 22, 23, 25, 34, 35, 36) est un oxymètre optique mettant en oeuvre au moins une première et une seconde sources lumineuses (21, 22), émettant respectivement à des première et seconde longueurs d'onde, et au moins un photodétecteur (23).

7. Dispositif de mesure selon l'une quelconque des revendications 1 à 6, **caractérisé en ce qu'**un desdits capteurs de mesure dudit deuxième ensemble de capteurs (14) est un oxymètre optique mettant en oeuvre au moins une diode électroluminescente (27), émettant à une première longueur d'onde, et au moins une photodiode (26).

8. Dispositif de mesure selon l'une quelconques des revendications 1 à 7, **caractérisé en ce que** ledit module (16) porte au moins un capteur de température (36).

9. Dispositif de mesure selon l'une quelconques des revendications 1 à 8, **caractérisé en ce que** ladite protubérance (13) comprend au moins deux électrodes (25) de mesure d'une résistance électrodermale.

10. Dispositif de mesure selon la revendication 9, **caractérisé en ce que** lesdits moyens de traitement comprennent des moyens de redressement, de filtrage passe bande et d'amplification d'au moins un signal issu desdites électrodes.

11. Dispositif de mesure selon la revendication 10, **caractérisé en ce que** lesdits moyens de traitement comprennent des moyens de suppression d'une composante continue dudit au moins un signal issu des électrodes.

12. Dispositif de mesure selon l'une quelconque des revendications 1 à 11, **caractérisé en ce que** lesdits moyens de traitement sont configurés pour délivrer au moins une information représentative d'au moins un paramètre physiologique appartenant au groupe comprenant :
- le pouls ;
- le taux d'oxygène dans le sang ;
- l'impédance de peau ;
- une grandeur fonction de l'impulsion cardiaque;
- le rythme respiratoire, l'arythmie respiratoire et/ou l'amplitude respiratoire ;
- la température cutanée ;
- la sudation au niveau du poignet ;
et les variations et/ou combinaisons de ces grandeurs.

13. Dispositif de mesure selon la revendication 12 quand elle dépend de l'une quelconque des revendications 8 à 11, **caractérisé en ce qu'**il comprend des moyens de transmission d'une information lorsqu'au moins un desdits capteurs de mesure constate une anomalie appartenant au groupe suivant :
- absence d'énergie au niveau desdits capteurs d'ondes de pression piézoélectriques (34, 35) ;
- impédance supérieure à un seuil prédéterminé desdites électrodes (25) de mesure de la résistance électrodermale ;
- variation supérieure à un seuil prédéterminé de la température indiquée par ledit au moins un capteur de température (36) ;
- absence de modulation de flux indiqués par ledit au moins photodétecteur (23).

14. Dispositif de mesure selon l'une quelconque des revendications 1 à 13, **caractérisé en ce que** ledit dispositif de mesure (10) est un dispositif ambulatoire adapté pour une utilisation portative.

## Claims

1. Device (10) for measurement of at least one physiological parameter in a living person, comprising a measurement module (16) which is fitted on a bracelet (11), the said module (16) supporting processing means which are supplied by at least one signal obtained from at least one measurement sensor which is designed to come into contact with the skin of the said person, at one of his wrists,
the said measurement module (16) comprising a first series of sensors (21, 22, 23, 25, 34, 35, 36) which are supported by a protuberance (13) which projects inside the said bracelet (11), and a second series of sensors (14) which are situated in the vicinity of the said protuberance (13), the form of the said protuberance (13) being defined such as to co-operate with the said wrist in order to prevent or limit rotation of the said bracelet (11) around the said wrist, and to place the said first series of sensors (21, 22, 23, 25, 34, 35, 36) and the said second series of sensors (14) at the radial artery of the said wrist, the said processing means comprising means for de-noising the signals obtained from the said first series of sensors (21, 22, 23, 25, 34, 35, 36) according to the signals obtained from the said second series of sensors (14),
**characterised in that** the sensors of the said first series of sensors comprise at least one optical sensor (21, 22, 23) and at least one pressure wave sensor (34, 35), such as to carry out measurements which are based on different physical principles and provide correlated measurement signals, and
**in that** the said processing means are supplied by the said correlated measurement signals obtained from the said optical sensor (21, 22, 23) and from the said pressure wave sensor (34, 35) of the said first series of sensors.

2. Device for measurement according to claim 1, **characterised in that** at least one of the said measurement sensors of the said first series of sensors (21, 22, 23, 25, 34, 35, 36) is a passive measurement sensor which carries out measurements continually.

3. Device for measurement according to one of claims 1 and 2, **characterised in that** the said protuberance (13) is fitted such as to be mobile relative to the said measurement module.

4. Device for measurement according to one of claims 1 to 3, **characterised in that** the said pressure wave sensor is a piezo-electric sensor which comprises two piezo-electric cells (34, 35) which are fitted in opposition on a plane which is tangent to the measurement module (16).

5. Device for measurement according to one of claims 1 to 4, **characterised in that** one of the said measurement sensors of the said second series of sensors (14) is an accelerometer.

6. Device for measurement according to any one of claims 1 to 5, **characterised in that** one of the said measurement sensors of the said first series of sensors (21, 22, 23, 25, 34, 35, 36) is an optical oxymeter which implements at least a first and a second source of light (21, 22) which emit respectively at first and second wave lengths, and at least one photodetector (23).

7. Device for measurement according to any one of claims 1 to 6, **characterised in that** one of the said measurement sensors of the said second series of sensors (14) is an optical oxymeter which implements at least one light-emitting diode (27) which emits at a first wave length, and at least one photodiode (26).

8. Device for measurement according to any one of claims 1 to 7, **characterised in that** the said module (16) supports at least one temperature sensor (36).

9. Device for measurement according to any one of claims 1 to 8, **characterised in that** the said protuberance (13) comprises at least two electrodes (25) for measurement of an electro-dermal resistance.

10. Device for measurement according to claim 9, **characterised in that** the said processing means comprise means for rectification, for pass-band filtering, and for amplification of at least one signal obtained from the said electrodes.

11. Device for measurement according to claim 10, **characterised in that** the said processing means comprise means for elimination of a continuous component of the said at least one signal obtained from the electrodes.

12. Device for measurement according to any one of claims 1 to 11, **characterised in that** the said processing means are configured to provide at least one item of data which is representative of at least one physiological parameter belonging to the group which comprises:
- the pulse;
- the level of oxygen in the blood;
- the impedance of the skin;
- a value which is dependent on the cardiac pulse;
- the respiratory rhythm, respiratory arrhythmia and/or respiratory amplitude;
- the temperature of the skin;
- the sweating on the wrist;
and the variations and/or combinations of these values.

13. Device for measurement according to claim 12 when it is dependent on any one of claims 8 to 11, **characterised in that** it comprises means for transmission of data when at least one of the said measurement sensors detects an abnormality which belongs to the following group:
- absence of energy at the said piezo-electric pressure wave sensors (34, 35);
- impedance greater than a predetermined threshold of the said electrodermal resistance measurement electrodes (25);
- variation greater than a predetermined threshold of the temperature indicated by the said at least one temperature sensor (36);
- absence of modulation of flows indicated by the said at least one photodetector (23).

14. Device for measurement according to any one of claims 1 to 13, **characterised in that** the said device (10) for measurement is an ambulatory device suitable for portable use.

## Patentansprüche

1. Vorrichtung (10) zur Messung mindestens eines physiologischen Parameters bei einer lebenden Versuchsperson, umfassend ein Messmodul (16), das auf einem Armband (11) angebracht ist, wobei das Modul (16) Behandlungsmittel trägt, die durch mindestens ein Signal gespeist werden, das aus mindestens einem Messwertsensor stammt, der bereitgestellt wird, um mit der Haut der Versuchsperson an einem ihrer Handgelenke in Berührung zu kommen,
wobei das Messmodul (16) eine Gruppe von Sensoren (21, 22, 23, 25, 34, 35, 36), die von einer Ausstülpung (13), die zum Innern des Armbandes (11) vorsteht, getragen werden, und eine zweite Gruppe von Sensoren (14), die sich in der Nähe der Ausstülpung (13) befinden, umfasst, wobei die Form der Ausstülpung (13) derart definiert ist, dass sie mit dem Handgelenk zusammenwirkt, um eine Drehung des Armbandes (11) um das Handgelenk zu verhindern oder einzuschränken und um die erste Gruppe von Sensoren (21, 22, 23, 25, 34, 35, 36) und die zweite Gruppe von Sensoren (14) an der Radialarterie des Handgelenks anzuordnen,
wobei die Behandlungsmittel Mittel zur Rauschunterdrückung der Signale, die aus der ersten Gruppe von Sensoren (21, 22, 23, 25, 34, 35, 36) stammen, in Abhängigkeit von den Signalen, die aus der zweiten Gruppe von Sensoren (14) stammen, umfassen,
**dadurch gekennzeichnet, dass** die Sensoren der ersten Gruppe von Sensoren mindestens einen optischen Sensor (21, 22, 23) und mindestens einen Druckwellensensor (34, 35) umfassen, um Messungen vorzunehmen, die auf unterschiedlichen physikalischen Grundlagen beruhen und korrelierte Messsignale abgeben,
dass die Behandlungsmittel durch die korrelierten Messsignale gespeist werden, die aus dem optischen Sensor (21, 22, 23) und dem Druckwellensensor (34, 35) der ersten Gruppe von Sensoren stammen.

2. Messvorrichtung nach Anspruch 1, **dadurch gekennzeichnet, dass** mindestens einer der Messsensoren der ersten Sensorgruppe (21, 22, 23, 25, 34, 35, 36) ein passiver Messwertsensor ist, der kontinuierlich Messungen durchführt.

3. Messvorrichtung nach einem der Ansprüche 1 und 2, **dadurch gekennzeichnet, dass** die Ausstülpung (13) im Verhältnis zum Messmodul beweglich angebracht ist.

4. Messvorrichtung nach einem der Ansprüche 1 bis 3, **dadurch gekennzeichnet, dass** der Druckwellensensor ein piezoelektrischer Sensor ist, der zwei piezoelektrische Zellen (34, 35) umfasst, die in einer Tangentenebene zum Messmodul (16) gegenüberliegend angebracht sind.

5. Messvorrichtung nach einem der Ansprüche 1 bis 4, **dadurch gekennzeichnet, dass** einer der Messwertsensoren der zweiten Sensorgruppe (14) ein Beschleunigungsmesser ist.

6. Messvorrichtung nach einem der Ansprüche 1 bis 5, **dadurch gekennzeichnet, dass** einer der Messsensoren der ersten Sensorgruppe (21, 22, 23, 25, 34, 35, 36) ein optischer Oxymeter ist, der mindestens eine erste und eine zweite Lichtquelle (21, 22), die jeweils auf ersten und zweiten Wellenlängen emittieren, und mindestens einen Photodetektor (23) umsetzt.

7. Messvorrichtung nach einem der Ansprüche 1 bis 6, **dadurch gekennzeichnet, dass** einer der Messwertsensoren der zweiten Sensorgruppe (14) ein optischer Oxymeter ist, der mindestens eine Leuchtdiode (27), die auf einer ersten Wellenlänge emittiert, und mindestens eine Photodiode (26) umsetzt.

8. Messvorrichtung nach einem der Ansprüche 1 bis 7, **dadurch gekennzeichnet, dass** das Modul (16) mindestens einen Temperatursensor (36) trägt.

9. Messvorrichtung nach einem der Ansprüche 1 bis 8, **dadurch gekennzeichnet, dass** die Ausstülpung (13) mindestens zwei Messelektroden (25) eines elektrodermalen Widerstandes umfasst.

10. Messvorrichtung nach Anspruch 9, **dadurch gekennzeichnet, dass** die Behandlungsmittel Mittel zum Gleichrichten, Bandpassfiltern und Verstärken mindestens eines Signals aus den Elektroden umfassen.

11. Messvorrichtung nach Anspruch 10, **dadurch gekennzeichnet, dass** die Behandlungsmittel Mittel zum Unterdrücken einer Gleichstromkomponente des mindestens einen Signals aus den Elektroden umfassen.

12. Messvorrichtung nach einem der Ansprüche 1 bis 11, **dadurch gekennzeichnet, dass** die Behandlungsmittel konfiguriert sind, um mindestens eine Information abzugeben, die mindestens einen physiologischen Parameter darstellt, der zu der Gruppe gehört, die Folgendes umfasst:
- den Puls;
- den Sauerstoffgehalt im Blut;
- die Impedanz der Haut;
- eine vom Herzschlag abhängige Größe;
- die Atemfrequenz, Atemstörung und/oder Atemamplitude;
- die Hauttemperatur;
- die Schweißabsonderung am Handgelenk;
und die Variationen und/oder Kombinationen dieser Größen.

13. Messvorrichtung nach Anspruch 12 in Abhängigkeit von einem der Ansprüche 8 bis 11, **dadurch gekennzeichnet, dass** sie Mittel zum Senden einer Information umfasst, wenn mindestens einer der Messwertsensoren eine Anomalie feststellt, die zu der folgenden Gruppe gehört:
- keine Energie an den piezoelektrischen Druckwellensensoren (34, 35);
- Impedanz höher als ein vorherbestimmter Schwellenwert der Messelektroden (25) des elektrodermalen Widerstands;
- Schwankung der Temperatur, die von dem mindestens einen Temperatursensor (36) angegeben wird, höher als ein vorherbestimmter Schwellenwert;
- keine Modulation der Flüsse, die von dem mindestens einen Photodetektor (23) angegeben werden.

14. Messvorrichtung nach einem der Ansprüche 1 bis 13, **dadurch gekennzeichnet, dass** die Messvorrichtung (10) eine ambulante Vorrichtung ist, die für eine tragbare Verwendung geeignet ist.
